# EUROPEAN PATENT APPLICATION

(11) **EP 4 091 633 A1**
(43) Date of publication of application: **23.11.2022**
(21) Application number: 21741964.7
(22) Date of filing: 15.01.2021
(51) Int. Cl.: A61K 45/00, A61K 39/395, A61P 3/10, A61P 9/00, A61P 25/28, A61P 43/00, C07K 16/28, C07K 16/46, C12N 15/13, C12N 15/62

(54) **PROPHYLACTIC OR THERAPEUTIC AGENT FOR DEMENTIA**

(30) Priority: 15.01.2020 JP 2020004403
(71) Applicant: OSAKA UNIVERSITY, Suita-shi Osaka 565-0871 (JP); Mitsubishi Tanabe Pharma Corporation, Osaka-shi, Osaka 541-8505 (JP)
(72) Inventor: YAMASHITA, Toshihide, Suita-shi, Osaka 565-0871 (JP); ITOKAZU, Takahide, Suita-shi, Osaka 565-0871 (JP); UNO, Hiroki, Suita-shi, Osaka 565-0871 (JP); ISHIDA, Hirokazu, Osaka-shi, Osaka 541-8505 (JP)
(74) Representative: Hoffmann Eitle
(86) International application number: PCT/JP2021/001276
(87) International publication number: WO 2021/145435

(57) **Abstract**

This invention provides an agent comprising a RGMa inhibiting substance for preventing or treating dementia selected from diabetic dementia and vascular dementia.

## Description

### TECHNICAL FIELD

The present invention relates to an agent comprising a RGMa inhibiting substance for preventing or treating diabetic dementia and vascular dementia.

### BACKGROUND ART

The number of patients with diabetes and dementia is increasing each year in step with the aging of the population, and it has become clear that those suffering from diabetes are at an increased risk of developing Alzheimer's disease or vascular dementia, and there is a close pathological relationship between the two diseases (Non Patent Document 1, and Non Patent Document 2). Further, there have been many reports, that even in a case where a diabetic patient has not complication of dementia, decrease in cognitive function is recognized as compared to non-diabetic patients. With respect to such cognitive dysfunction of diabetic patient, decrease in attention or concentration, decrease in visual memory or verbal memory, or declined rating in the mini-mental state examination (MMSE) are reported (Non Patent Document 3).

Recently, a clinical disease type termed diabetic dementia, in which glucose metabolism abnormalities are intimately involved in development of dementia, has been proposed as a class in the classification of dementia with diabetes in addition to Alzheimer's disease and vascular dementia, (Non Patent Document 4, and Non Patent Document 5). Although diabetic dementia is less likely to present brain image findings characteristic to Alzheimer's disease (e.g., atrophy of the hippocampus), there appear more commonly complications of vascular lesions such as microinfarct lesions (Non Patent Document 6). Clinically, they are characterized in that the patients are slightly aged, and poorly controlled in diabetes, and decrease in attention and concentration, and impairment in executive function are noticeable rather than memory impairment, and their progression is slightly slow.

On the other hand, vascular dementia is dementia mainly caused by cerebrovascular disorder, and particularly defined as dementia which is mainly caused by a cerebral small vessel disease such as Binswanger's disease, and multiple lacunar infarcts, and in which there are (1) dementia, (2) cerebrovascular disorder, and (3) cause-and-effect relationship between the two.

The clinical diagnostic criteria of NINDS-AIREN (National Institute of Neurological Disorders and Stroke - Association International pour la Recherche et l'Enseignement en Neurosciences) give five disease type classes of (1) multiple infarct type, (2) single lesion type, (3) small vessel lesion type, (4) hypoperfusion type, and (5) cerebral hemorrhage type. However, since there is a problem that these disease types are etiologically and clinically uneven to each other, it is understood that vascular dementia is a heterogeneous disease concept that includes various pathological states (Non Patent Document 7, and Non Patent Document 8).

It is also widely known that diabetes is one of the risk factors for vascular dementia (Non Patent Document 8, and Non Patent Document 9).

RGM (repulsive guidance molecule) is a membrane protein that has been initially identified as an axon guidance molecule in the visual system (see Non Patent Document 10). RGM family includes three members called RGMa, RGMb, and RGMc (Non Patent Document 11). At least RGMa and RGMb are known to work in the same signaling mechanism (Non Patent Document 12). RGMc plays an important role in iron metabolisms.

Subsequent studies have revealed that RGM functions to control, for example, axon guidance and laminar formation in *Xenopus* and chick embryos, and cephalic neural tube closure in mouse embryos (Non Patent Document 13). Patent Document 1 discloses an axon regeneration promoting agent containing an anti-RGM neutralizing antibody as an active ingredient.

In addition to its functions in developmental stages, RGMa is expressed again after central nervous system injuries in an adult human and rat. Further, inhibition of RGMa in rat promotes axon growth after spinal cord injuries and facilitates functional recovery (Non Patent Document 14). From these facts, RGMa is considered as an inhibitor of axon regeneration after central nervous system injuries. Specific examples of antibodies to neutralize RGMa include those described in Patent Document 2 (such as 5F9, and 8D1), Patent Document 3 (such as AE12-1, an AE12-1Y), and Patent Document 4 (such as r116A3, r70E4, r116A3C, and rH116A3).

Patent Document 2 discloses the therapeutic use of an anti-RGMa antibody for dementia.

As described above, roles of RGMa in central nervous system injuries have been reported, however, involvement of RGMa in the treatment of diabetic dementia and vascular dementia, has not been identified and no such therapeutic agent has been known.

### CITATION LIST

### Patent Document

[Patent Document 1] International Publication No. WO2005/087268
[Patent Document 2] International Publication No. WO2009/106356
[Patent Document 3] International Publication No. WO2013/112922
[Patent Document 4] International Publication No. WO2016/175236

### Non Patent Document

[Non Patent Document 1] Neurology, 45: 1161, 1995
[Non Patent Document 2] Am. J. Epidemiol., 1455: 301, 1997
[Non Patent Document 3] Diabetes Care, 20: 438, 1997
[Non Patent Document 4] Dement Geriatr Cogn Disord, 35: 280-290, 2013
[Non Patent Document 5] J. Neurol. Sci., 349: 45-51, 2015
[Non Patent Document 6] Nat Rev Neurol 5: 305-306, 2009
[Non Patent Document 7] Neurology, 43: 250-260, 1993
[Non Patent Document 8] Guideline for the Treatment of Dementia 2017, Chapter 14: Vascular Dementia
[Non Patent Document 9] Neuron, 83: 844-866, 2013
[Non Patent Document 10] Neuron 5, 735-743 (1990).
[Non Patent Document 11] Philos. Trans. R. Soc. Lond. B Biol. Sci., 361: 1513-29, 2006
[Non Patent Document 12] Biochem. Biophys. Res. Commun., 382, 795-800 (2009).
[Non Patent Document 13] Curr. Opin. Neurobiol., 17, 29-34 (2007).
[Non Patent Document 14] J. Cell Biol., 173, 47-58 (2006).

### SUMMARY OF INVENTION

### Problem to be solved by the invention

An object of the present invention is to provide an effective drug for diabetic dementia and vascular dementia.

### Means to solve the problem

The present inventors have intensively studied for achieving the above object and found that a RGMa inhibiting substance, in particular, an anti-RGMa neutralizing antibody has an improving effect on diabetic dementia and vascular dementia, thereby completed the present invention.

The present invention is as follows.
[1] An agent for preventing or treating dementia selected from diabetic dementia and vascular dementia comprising a RGMa inhibiting substance.
[2] An agent for preventing or treating diabetic dementia comprising a RGMa inhibiting substance.
[3] An agent for preventing or treating vascular dementia comprising a RGMa inhibiting substance.
[4] The preventive or therapeutic agent according to any one of [1] to [3], wherein the RGMa inhibiting substance is an anti-RGMa neutralizing antibody.
[5] The preventive or therapeutic agent according to [4], wherein the anti-RGMa neutralizing antibody is a humanized antibody.
[6] The preventive or therapeutic agent according to [4] or [5], wherein the anti-RGMa neutralizing antibody is an antibody recognizing an amino acid sequence selected from SEQ ID NOS: 16, 36, 37, 38 and 39.
[7] The preventive or therapeutic agent according to any one of [4] to [6], wherein the anti-RGMa neutralizing antibody is an antibody selected from the following (a) to (l):
   (a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6, and an LCDR3 comprising the amino acid sequence represented by SEQ ID NO: 7, and a heavy chain variable region comprising an HCDR1 comprising the amino acid sequence represented by SEQ ID NO: 8, an HCDR2 comprising the amino acid sequence represented by SEQ ID NO: 9, and an HCDR3 comprising the amino acid sequence represented by SEQ ID NO: 10;
   (b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12, and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15, and an HCDR3 comprising an amino acid sequence comprising SFG;
   (c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18, and an LCDR3 comprising the amino acid sequence represented by SEQ ID NO: 19, and a heavy chain variable region comprising an HCDR1 comprising the amino acid sequence represented by SEQ ID NO: 20, an HCDR2 comprising the amino acid sequence represented by SEQ ID NO: 21, and an HCDR3 comprising the amino acid sequence represented by SEQ ID NO: 22;
   (d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24, and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ ID NO: 27, and an HCDR3 comprising the amino acid sequence represented by SEQ ID NO: 28;
   (e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 42, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 43, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34; and
   (l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34.
[8] A method of preventing or treating dementia selected from diabetic dementia and vascular dementia, which comprises administration of an effective dose of a RGMa inhibiting substance to a mammal in need of treatment.
[9] The preventive or therapeutic method according to [8], wherein the RGMa inhibiting substance is an anti-RGMa neutralizing antibody.
[10] Use of a RGMa inhibiting substance in manufacture of an agent for preventing or treating dementia selected from diabetic dementia and vascular dementia.
[11] The use according to [10], wherein the RGMa inhibiting substance is an anti-RGMa neutralizing antibody.

### Effects of Invention

According to the present invention, a RGMa inhibiting substance, in particular. an anti-RGMa neutralizing antibody, is useful as an agent for preventing or treating dementia selected from diabetic dementia and vascular dementia.

### BRIEF DESCRIPTION OF THE DRAWINGS

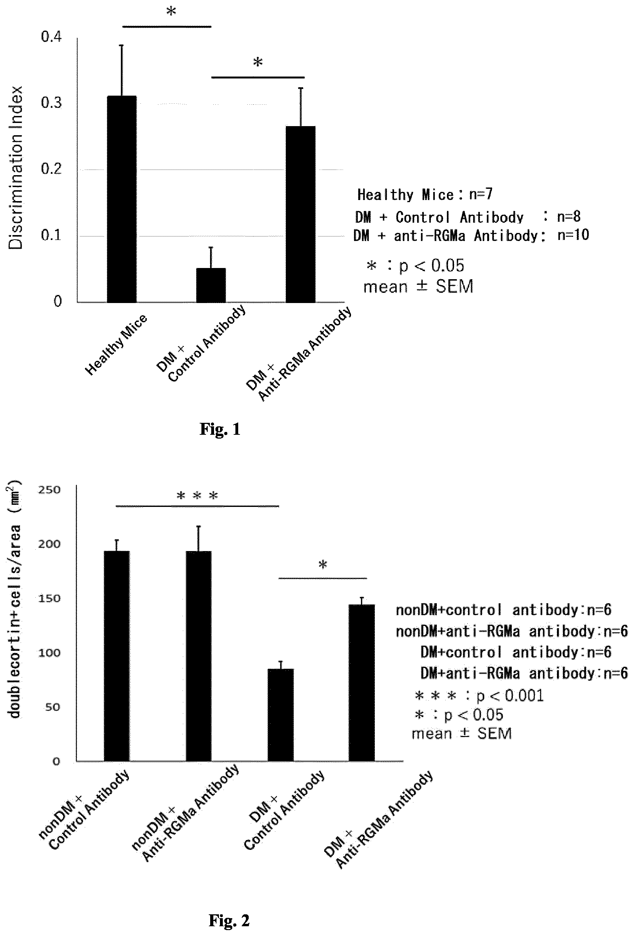

Fig. 1 is a graph showing the Discrimination Index (DI) values obtained from the anti-RGMa neutralizing antibody(may be simply referred to as anti-RGMa antibody)-administered group and the isotype control antibody-administered group at 4 weeks after the onset of pathological conditions of diabetes (DM), respectively, as well as the DI value of healthy mice.
Fig. 2 is a graph showing the numbers of doublecortin positive cells upon administration of the anti-RGMa neutralizing antibody or the control antibody to non-diabetic (non-DM) mice or diabetic (DM) mice. The numbers of doublecortin positive cells were normalized by the area of the hippocampal dentate gyrus.

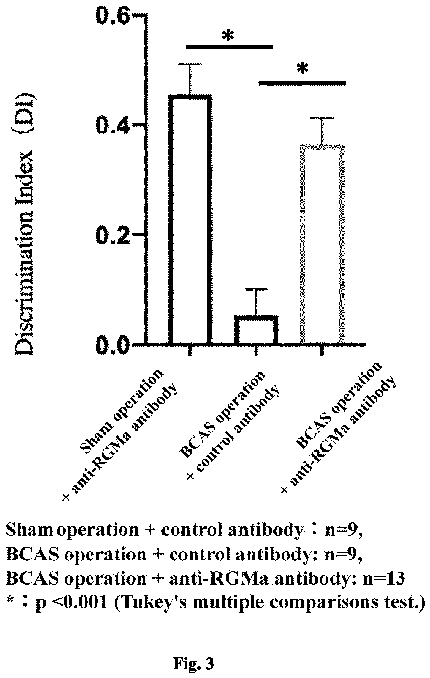

Fig. 3 is a graph showing the Discrimination Index (DI) values obtained respectively from the anti-RGMa neutralizing antibody-administered group and the isotype control antibody-administered group of chronic cerebral hypoperfusion model, as well as the DI value obtained from the isotype control antibody-administered group of Sham mice.

### DETAILED DESCRIPTION OF THE INVENTION

The terms used in the present invention will be described below.

### [Neutralization]

The term "neutralization" as used herein refers to an action of binding to an objective target and inhibiting functions of the target. For example, a RGMa inhibiting substance refers to a substance which binds to RGMa and consequently acts to inhibit a biological activity of RGMa.

### [Epitope]

As used herein, the term "epitope" includes a polypeptide determinant that can specifically bind to an immunoglobulin or a T-cell receptor. In some embodiments, an epitope can include a chemically active group on the surface of the molecule (for example, an amino acid, a sugar side chain, phosphoryl or sulfonyl). In some embodiments, an epitope can have particular characteristics of three-dimensional structure and/or electric charge. Epitopes refer to regions in antigens, to which antibodies bind.

### [Isolated]

As used herein, the term "isolated" such as in isolated RGMa inhibiting substance (for example, antibody) means being identified, and separated and/or recovered from components in natural states. Impurities in natural states are substances that can interfere with the diagnostic or therapeutic use of the antibody, including enzymes, hormones and other proteinous or nonproteinous solutes. Generally, RGMa inhibiting substances or the like may be isolated through at least one purification step. RGMa inhibiting substances purified through at least one purification step can be referred to as "isolated RGMa inhibiting substances".

### [Antibody]

As used herein, the term "antibody" refers broadly to an immunoglobulin (Ig) molecule comprising four polypeptide chains, namely two heavy chains (H chains) and two light chains (L chains), that substantially retain the characteristics of an Ig molecule to bind to an epitope.

### [Human Antibody]

As used herein, the term "human antibody" refers to an antibody comprising light and heavy chains which are both derived from human immunoglobulins. Depending on the difference in the constant region of the heavy chain, human antibodies include IgG comprising a γ heavy chain (including IgG1, IgG2, IgG3 and IgG4); IgM comprising a µ heavy chain; IgA comprising an α heavy chain (including IgA1 and IgA2); IgD comprising a δ heavy chain; and IgE comprising an ε heavy chain. In principle, a light chain comprises either κ or λ chain.

### [Humanized Antibody]

As used herein, the term "humanized antibody" refers to an antibody comprising variable regions comprising complementarity determining regions from antibodies derived from non-human animals and framework regions derived from human antibodies, and constant regions derived from human antibodies.

### [Chimeric Antibody]

As used herein, the term "chimeric antibody" refers to an antibody in which the light chain, the heavy chain, or both comprise a non-human derived variable region and a human derived constant region.

### [Monospecific Antibody]

As used herein, the term "monospecific antibody" refers to an antibody comprising a single independent antigen recognition site having a single antigen specificity. For example, a monospecific antibody that recognizes RGMa may be referred herein to as a RGMa-monospecific antibody.

### [Multispecific Antibody]

As used herein, the term "multispecific antibody" refers to antibodies comprising two or more independent antigen recognition sites having two or more different antigen specificities, including bispecific antibodies having two antigen specificities and trispecific antibodies having three antigen specificities.

### [Complementarity Determining Region (CDR)]

The term "complementarity determining region (CDR)" refers to a region forming an antigen binding site in a variable region of an immunoglobulin molecule, which is also called a hypervariable region, and particularly refers to a portion in which the amino acid sequence changes greatly for each immunoglobulin molecule. As CDRs, light and heavy chains each have three CDRs. Three CDRs contained in a light chain may be referred to as LCDR1, LCDR2, and LCDR3, while three CDRs contained in a heavy chain may be referred to as HCDR1, HCDR2, and HCDR3. For example, CDRs of an immunoglobulin molecule are assigned according to the Kabat numbering system (Kabat, et al., 1987, Sequences of Proteins of Immunological Interest, US Department of Health and Human Services, NIH, USA).

### [Effective Dose]

The term "effective dose" refers to a sufficient dose of a preventive or therapeutic agent to alleviate or ameliorate the severity and/or duration of a disorder, or one or more symptoms thereof; to prevent progression of the disorder; to reverse the disorder; to prevent the recurrence, onset, development, or progression of one or more symptoms associated with the disorder; to detect the disorder; or to enhance or improve one or more preventive or therapeutic effects of another therapy (for example, a prophylactic drug or a therapeutic drug).

### [Percent (%) Identity of Amino Acid Sequence]

"Percent (%) identity" of the amino acid sequence of a candidate polypeptide sequence, such as a variable region, with respect to the amino acid sequence of a reference polypeptide sequence is defined as a percent of the same amino acid residues in the candidate sequence as the amino acid residues in the particular reference polypeptide sequence, which percent is obtained by arranging the sequences and introducing gaps if necessary to obtain maximal % identity, without considering any conservative substitutions as part of the sequence identity. Alignment for determination of % identity can be accomplished by using various methods within the skill of the art, for example, using a publicly available computer software, such as BLAST, BLAST-2, ALIGN, or Megalign (DNASTAR) software. Those skilled in the art can determine appropriate parameters for sequence alignment, including algorithm needed to achieve maximal alignment over the full length of the sequence to be compared. However, for the purposes herein, values of % identity are obtained in pairwise alignment using a computer program for comparing sequences, BLAST.

When BLAST is used in comparison of amino acid sequences, the % identity of a given amino acid sequence A to a given amino acid sequence B is calculated as follows:
a fraction X/Y multiplied by 100
where X is the number of amino acid residues scored as identical in a programmed alignment of A and B by the sequence alignment program Blast, and Y is the total number of amino acid residues in B. It will be understood that when the lengths of amino acid sequences A and B are different, the % identities of A to B and of B to A are different. Unless stated otherwise, all % identity values herein are obtained using a computer program BLAST, as described in the immediately preceding paragraph.

### [Conservative Substitution]

"Conservative substitution" means replacement of an amino acid residue with another chemically similar amino acid residue so as not to substantially alter the activity of the peptide. Examples thereof include a case where a hydrophobic residue is substituted with another hydrophobic residue, and a case where a polar residue is substituted with another polar residue having the same charge. Examples of functionally similar amino acids eligible for such substitution include, as for non-polar (hydrophobic) amino acids, alanine, valine, isoleucine, leucine, proline, tryptophan, phenylalanine, and methionine. As for polar (neutral) amino acids, they include glycine, serine, threonine, tyrosine, glutamine, asparagine, and cysteine. As for positively charged (basic) amino acids, they include arginine, histidine, and lysine. As for negatively charged (acidic) amino acids, they include aspartic acid, and glutamic acid.

The present invention will be described in detail below.

The present invention provides an agent for preventing or treating dementia selected from diabetic dementia and vascular dementia, which is a novel use of a RGMa inhibiting substance.

Further, the present invention provides a method of preventing or treating dementia selected from diabetic dementia and vascular dementia, comprising a step of administering a preventive or therapeutic agent comprising an effective dose of a RGMa inhibiting substance to a mammal in need of treatment.

### <RGMa Inhibiting substance>

There is no particular restriction on the RGMa inhibiting substance of the present invention, insofar as it is a substance that acts on RGMa itself to inhibit or reduce the activity of RGMa (hereinafter sometimes referred to simply as "RGMa activity"). For example, a substance having an activity that directly inhibits (or reduces) the RGMa activity by binding to the RGMa, or an activity that indirectly inhibits (or reduces) the RGMa activity by inhibiting the binding of a RGMa to a receptor (e.g., the compound, antibody, etc. described below) is referred to as the RGMa inhibiting substance of the present invention.

The RGMa inhibiting substance of this invention may also be a substance that inhibits the expression of RGMa, for example, a substance that inhibits the expression of RGMa to inhibit (reduces) the RGMa activity (e.g., a nucleic acid molecule described below) is also included in the RGMa inhibiting substance of the present invention.

RGMa is identified as a protein that inhibits neurite outgrowth in the central nervous system. A human RGMa protein is biosynthesized as a precursor protein comprising 450 amino acids as shown in SEQ ID NO: 1. The signal peptide from Met 1 to Pro 47 present at the N terminus (which refers to the peptide from the methionine residue at position 1 to the proline residue at position 47 counted from the N-terminal side, and is hereafter described as above) is removed. Then, the peptide bond between Asp 168 and Pro 169 is cleaved to generate an N-terminal domain. In the C-terminal fragment from Pro 169, the peptide from Ala 425 to Cys 450 at the C terminus is removed so that Ala 424 becomes the C terminus. Then, a GPI anchor is added to the C-terminal carboxyl group of Ala 424 to generate a C-terminal domain. The human RGMa protein is expressed on the cell membrane via a GPI anchor as a mature protein in which the N-terminal domain (Cys 48 to Asp 168) and the C-terminal domain (Pro 169 to Ala 424) are linked by a disulfide bond.

RGMa in the present invention may be derived from any animals. Preferably, RGMa is human RGMa. A human RGMa precursor protein comprises the amino acid sequence shown in SEQ ID NO: 1 in Sequence Listing. A mouse RGMa precursor protein comprises the amino acid sequence shown in SEQ ID NO: 2 in Sequence Listing, while a rat RGMa precursor protein comprises the amino acid sequence shown in SEQ ID NO: 3 in Sequence Listing. Removal of the C-terminal peptides from each amino acid sequence results in mature proteins having the same amino acid sequence, respectively.

RGMa genes include, but are not limited to, a human RGMa gene comprising the nucleotide sequence shown in SEQ ID NO: 4. Nucleotide sequences of RGM genes derived from various organisms can be easily obtained from known databases (for example, GenBank).

Specific examples of the RGMa inhibiting substance of the present invention include low molecular weight compounds, anti-RGMa neutralizing antibodies, and functionally modified antibodies thereof, conjugated antibodies thereof, and antigen-binding fragments thereof, as well as an siRNA (short interfering RNA), an shRNA (short hairpin RNA), and an antisense oligonucleotide, which are nucleic acid molecules of RGMa. Among these RGMa inhibiting substances, an anti-RGMa neutralizing antibody, a functionally modified antibody thereof, a conjugated antibody thereof, and an antigen-binding fragment thereof are preferable, an anti-RGMa neutralizing antibody, and an antigen-binding fragment thereof are more preferable, and an anti-RGMa neutralizing antibody is especially preferable.

### <Anti-RGMa Neutralizing Antibody>

According to the present invention, the anti-RGMa neutralizing antibody is an antibody that binds to RGMa to neutralize the RGMa activity, and may be a polyclonal, or monoclonal antibody. A monoclonal antibody is more preferable according to the present invention. The anti-RGMa-neutralizing antibody of the present invention may be a RGMa-monospecific antibody, or a multispecific antibody that recognizes RGMa and a plurality of other antigens. A RGMa-monospecific antibody is more preferable.

Specifically, the epitopes in human RGMa are preferably one or more of SEQ ID NO: 16 (amino acid numbers 47 to 69 in SEQ ID NO: 1), SEQ ID NO: 36 (amino acid numbers 298 to 311 in SEQ ID NO: 1), SEQ ID NO: 37 (amino acid numbers 322 to 335 in SEQ ID NO: 1), SEQ ID NO: 38 (amino acid numbers 349 to 359 in SEQ ID NO: 1), and SEQ ID NO: 39 (amino acid numbers 367 to 377 in SEQ ID NO: 1); more preferably a combination of SEQ ID NOS: 36 and 37;, and particularly preferably a combination of SEQ ID NOS: 36, 37 and 39.

The anti-RGMa neutralizing antibodies of the present invention include polyclonal or monoclonal antibodies obtained by immunizing mammals such as mice with an antigen which is a RGMa protein or a partial fragment thereof (for example, epitope fragment described above); chimeric antibodies and humanized antibodies produced by gene recombination technology; and human antibodies produced, for example, by a transgenic animal producing human antibody. When the antibody of the present invention is administered to a human as a medicine, the antibody is desirably a humanized antibody or a human antibody from the viewpoint of reducing side effects.

Specific examples of the anti-RGMa neutralizing antibody of the present invention include the following antibodies (a) to (1). As the respective production methods therefor, the methods described in Patent Documents 2 to 4 may be used.

Examples thereof include antibodies to be selected from:
(a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6 and an LCDR3 comprising the amino acid sequence represented by SEQ ID NO: 7, and a heavy chain variable region comprising an HCDR1 comprising the amino acid sequence represented by SEQ ID NO: 8, an HCDR2 comprising the amino acid sequence represented by SEQ ID NO: 9 and an HCDR3 comprising the amino acid sequence represented by SEQ ID NO: 10 (the anti-RGMa neutralizing antibody also includes antibodies whose epitopes are the amino acid sequences represented by SEQ ID NOS: 36, 37 and 39);
(b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12 and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15 and an HCDR3 comprising an amino acid sequence comprising SFG (the anti-RGMa neutralizing antibody also includes antibodies whose epitopes are the amino acid sequences represented by SEQ ID NOS: 36, 37 and 38);
(c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18 and an LCDR3 comprising the amino acid sequence represented by SEQ ID NO: 19, and a heavy chain variable region comprising an HCDR1 comprising the amino acid sequence represented by SEQ ID NO: 20, an HCDR2 comprising the amino acid sequence represented by SEQ ID NO: 21 and an HCDR3 comprising the amino acid sequence represented by SEQ ID NO: 22;
(d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24 and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ ID NO: 27 and an HCDR3 comprising the amino acid sequence represented by SEQ ID NO: 28;
(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);
(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);
(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);
(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);
(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 42, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);
(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 43, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16);
(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16); and
(l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is the amino acid sequence represented by SEQ ID NO: 16).

Among these the antibody according to (a) is particularly preferable.

The anti-RGMa neutralizing antibodies of the present invention can be produced using production methods that are conventionally and commonly used. Antigens may be directly used for immunization, or may be used as a complex with a carrier protein. For preparing a complex of an antigen and a carrier protein, condensing agents such as glutaraldehyde, carbodiimides, and maleimide active esters can be used. Examples of the carrier protein include bovine serum albumin, thyroglobulin, hemocyanin, and KLH.

Examples of the mammal to be immunized include mice, rats, hamsters, guinea pigs, rabbits, cats, dogs, pigs, goats, horses and cattle. Examples of the inoculation method include subcutaneous, intramuscular and intraperitoneal administrations. When administered, antigens may be administered in mixture with complete or incomplete Freund's adjuvant, and are usually administered once every 2 to 5 weeks. Antibody-producing cells obtained from the spleen or lymph nodes of the immunized animals are fused with myeloma cells and isolated as hybridomas. As the myeloma cells, those derived from mammals such as mouse, rat, and human are used.

### <Polyclonal Antibody>

The polyclonal antibodies can be obtained, for example, from a serum obtained from an immunized animal which is the mammal as described above immunized with the antigen as described above, if necessary in combination with a Freund's adjuvant.

### <Monoclonal Antibody>

Specifically, the monoclonal antibodies can be obtained, for example, as follows. That is, the antigen as described above is used as an immunogen, and the immunogen is injected or transplanted once or several times in combination with a Freund's adjuvant, as necessary, to the mammal as described above subcutaneously, intramuscularly, intravenously, into a footpad, or intraperitoneally for immunization. Typically, the immunization is performed about once to four times every 1 day to 14 days from the initial immunization, and after about 1 day to 5 days from the final immunization, antibody-producing cells are obtained from the immunized mammal.

The monoclonal antibodies can be obtained by a method well known to a person skilled in the art (for example, "Current Protocols in Molecular Biology" (John Wiley & Sons (1987)), or Antibodies: A Laboratory Manual, Ed. Harlow and David Lane, Cold Spring Harbor Laboratory (1988)).

The "hybridomas" secreting monoclonal antibodies can be prepared according to the method of Köhler and Milstein, et al. (Nature, 256, 495, 1975) or a modified method based on it. That is, they are prepared by cell fusion between an antibody-producing cell included in the spleen etc. obtained from an immunized mammal, and a myeloma cell, which is not capable of producing an autoantibody, and derived from a mammal, preferably mouse, rat or human.

Examples of the myeloma cell which can be used in the cell fusion include mouse-derived myelomas such as P3/X63-AG8.653 (653), P3/NSI/1-Ag4-1 (NS-1), P3/X63-Ag8.U1 (P3U1), SP2/0-Ag14 (Sp2/O, Sp2), PAI, F0 and BW5147; rat-derived myelomas such as 210RCY3-Ag.2.3.; and human-derived myelomas such as U-266AR1, GM1500-6TG-A1-2, UC729-6, CEM-AGR, D1R11 and CEM-T15.

Examples of the fusion promoter include polyethylene glycols. The cell fusion can usually be performed by a reaction in a temperature range from 20°C to 40°C, preferably from 30°C to 37°C, for about 1 minute to 10 minutes usually using a polyethylene glycol (with an average molecular weight from 1000 to 4000) with a concentration from about 20% to 50%, wherein the ratio of the number of antibody-producing cells to the number of myeloma cells is usually from about 1:1 to 10:1

Screening of hybridoma clones producing the monoclonal antibodies can be carried out by culturing the hybridomas, for example, in a microtiter plate and assaying the culture supernatants in the wells for the reactivity against an immunogen by an immunochemical method such as ELISA.

In the screening of antibody-producing hybridomas, whether the antibody inhibits the RGMa activity in the present invention is also determined in addition to the binding assay with a RGMa protein. The screening methods allow for selection of the anti-RGMa neutralizing antibody of the present invention.

Clones can be further obtained from the wells containing hybridomas producing the desired antibodies by cloning with limiting dilution. Hybridomas are usually selected and grown in an animal cell culture medium containing 10% to 20% fetal bovine serum, supplemented with HAT (hypoxanthine, aminopterin and thymidine).

The monoclonal antibodies can be produced from the hybridomas by culturing the hybridomas *in vitro,* or growing them *in vivo,* for example, in ascitic fluids of mammals such as mice and rats, and isolating the monoclonal antibodies from the resulting culture supernatants or the ascitic fluids of the mammals.

When cultured *in vitro,* it is possible to use a nutrient medium suitable for growing, maintaining, and conserving hybridomas corresponding to various conditions such as the characteristics of the cell species to be cultured, or the culturing method, and producing a monoclonal antibody in the culture supernatant. Examples of the nutrient medium may include a known nutrient medium, or a nutrient medium prepared from a basal medium.

Examples of the basal medium include low-calcium media such as Ham's F12 medium, MCDB 153 medium, and low-calcium MEM medium, and high-calcium media such as MCDB 104 medium, MEM medium, D-MEM medium, RPMI 1640 medium, ASF 104 medium, and RD medium. The basal medium can contain, for example, sera, hormones, cytokines and/or various inorganic or organic substances according to the purpose.

The monoclonal antibodies can be isolated and purified by, for example, subjecting the above-described culture supernatant or ascitic fluid to saturated ammonium sulfate, euglobulin precipitation, a caproic acid treatment, a caprylic acid treatment, an ion exchange chromatography (such as DEAE or DE52), or an affinity column chromatography, for example with an anti-immunoglobulin column or a protein A column. Specifically, the monoclonal antibodies may be purified by any method known as an immunoglobulin purification method, and the purification can be easily achieved by means such as ammonium sulfate fractionation, PEG fractionation, ethanol fractionation, a method utilizing an anion exchanger, and further an affinity chromatography using a RGMa protein.

The monoclonal antibodies can also be obtained by a phage display method. In a phage display method, phages selected from an optional phage antibody library are screened using a desired immunogen, and phages having a desired binding ability to the immunogen are selected. Next, the antibody-corresponding sequence contained in the phage is isolated or sequenced. Based on the information of the isolated sequence or the determined sequence by the sequencing, an expression vector comprising a nucleic acid molecule encoding the antibody or antigen binding domain is constructed. The expression vector is then transfected into a cell line, and the cell line can be cultured to produce a monoclonal antibody. When a human antibody library is used as the phage antibody library, a human antibody having a desired binding ability can be produced.

### <Nucleic Acid Molecule>

A nucleic acid molecule encoding an anti-RGMa neutralizing antibody of the present invention or an antigen-binding fragment thereof can be obtained, for example, by the following method. First, total RNA is prepared from a cell such as hybridoma using a commercially available RNA extraction kit, and subsequently cDNAs are synthesized with a reverse transcriptase using random primers and the like. Next, by a PCR method using, as primers, oligonucleotides of sequences respectively conserved in the variable regions in the known human antibody heavy chain and light chain genes, cDNAs encoding the antibody are amplified. Sequences encoding the constant regions can be obtained by amplification of known sequences by a PCR method. The nucleotide sequence of the DNA can be sequenced by a conventional method, for example, by incorporating it into a plasmid for sequencing.

Alternatively, a DNA encoding the monoclonal antibody of the present invention can also be obtained by chemically synthesizing sequences of the variable regions or parts thereof and joining them to sequences comprising the constant regions.

The nucleic acid molecule may encode all of the constant regions and the variable regions of heavy and light chains, or may encode only the variable regions of heavy and light chains. In the case of encoding all of the constant regions and the variable regions, the nucleotide sequences of the constant regions of heavy and light chains are preferably those described in Nucleic Acids Research, vol. 14, p1779, 1986; The Journal of Biological Chemistry, vol. 257, p1516, 1982; or Cell, vol. 22, p197, 1980.

### <Functionally Modified Antibody>

A functionally modified antibody of an anti-RGMa neutralizing antibody is prepared by the following method. For example, when an anti-RGMa neutralizing antibody under this application is produced using CHO cells as host cells, in which the α1,6-fucosyl transferase (FUT8) gene is destructed, the fucose content in the sugar chain is decreased, and an antibody with an increased cell killing function is obtained. Meanwhile, when the same is produced using CHO cells as host cells, in which the FUT8 gene is transduced an antibody with a weak cell killing function is obtained (International Publication Nos. WO2005/035586, WO2002/31140, and WO00/61739). Meanwhile, the complement activation function can be modulated by modifying the amino acid residues in the Fc region (U.S. Patent Nos. 6,737,056, 7,297,775, and 7,317,091). Further, when the variant of the Fc region with enhanced binding to FcRn, which is one of Fc receptors, is used, the half-life in blood can be prolonged (Hashiguchi Shuhei, et al, Seikagaku, 2010, Vol. 82 (8), p710). These functionally modified antibodies can be produced by genetic engineering.

### <Conjugated Antibody>

Examples of a modified molecule of the anti-RGMa neutralizing antibody of the present invention include a conjugated antibody. Examples of the conjugated antibody include a conjugated antibody in which an anti-RGMa neutralizing antibody is bound chemically or by a genetic engineering technique to a functional molecule other than the present anti-RGMa neutralizing antibody such as nonpeptidic polymers such as polyethylene glycol (PEG), radioactive substances, toxins, low molecular weight compounds, cytokines, growth factors (such as TGF-β, NGF, and neurotrophin), albumins, enzymes, and other antibodies.

When PEG is bound as the functional molecule, PEG having, but without limitation, a molecular weight from 2,000 Da to 100,000 Da, more preferably from 10,000 Da to 50,000 Da can be used. PEG may be linear or branched. PEG can be bound to the N-terminal amino group in the amino acids of an anti-RGMa neutralizing antibody, etc., for example by using an NHS active group.

When a radioactive substance is used as the functional molecule, its examples include ¹³¹I, ¹²⁵I, ⁹⁰Y, ⁶⁴Cu, ⁹⁹Tc, ⁷⁷Lu, and ²¹¹At. The radioactive substance can be directly bound to the anti-RGMa neutralizing antibody by a chloramine-T method or the like.

When a toxin is used as the functional molecule, examples thereof include bacterial toxins (such as diphtheria toxin), phytotoxins (such as ricin), low molecular weight toxins (such as geldanamycin), maytansinoids and calicheamicins.

When a low molecular weight compound is used as the functional molecule, examples include daunomycin, doxorubicin, methotrexate, mitomycin, neocarzinostatin, vindesine, and fluorescent dyes such as FITC.

When an enzyme is used as the functional molecule, examples include luciferases (such as firefly luciferases, and bacterial luciferases; US Patent No. 4,737,456), malate dehydrogenases, ureases, peroxidases (such as horseradish peroxidase (HRPO)), alkaline phosphatases, β-galactosidases, glucoamylases, lysozymes, saccharide oxidases (such as glucose oxidase, galactose oxidase, and glucose-6-phosphate dehydrogenase), heterocyclic oxidases (such as uricase, and xanthine oxidase), lactoperoxidases, and microperoxidases.

Examples of linkers used for chemically bonding the toxin, low molecular weight compound, or enzyme include divalent radicals (such as alkylene, arylene, and heteroarylene), linkers represented by -(CR₂)ₙO(CR₂)ₙ- (wherein R is any substituent, and n is a positive integer), alkoxy repeating units (such as polyethyleneoxy, PEG, and polymethyleneoxy), alkylamino (such as polyethyleneamino, and Jeffamine^{™}), and diacid esters and amides (including succinate, succinamide, diglycolate, malonate, and caproamide). Chemical modification methods for binding functional molecules have already been established in this field (D. J. King., Applications and Engineering of Monoclonal Antibodies., 1998, T.J. International Ltd, Monoclonal Antibody-Based Therapy of Cancer., 1998, Marcel Dekker Inc.; Chari, et al., Cancer Res., 1992 Vol. 152: 127; and Liu, et al., Proc Natl Acad Sci USA., 1996 Vol 93: 8681).

### <Antigen-binding Fragment>

In embodiments of the present invention, "antigen-binding fragments" of antibodies means partial regions having antigen binding properties derived from the antibodies as described above. Specific examples of the fragments include F(ab')₂, Fab', Fab, Fv (variable fragment of antibody), disulfide-linked Fv, single-chain antibodies (scFv), and polymers thereof. Examples of the antigen-binding fragments also include conjugated fragments that bind, chemically or by genetic engineering, functional molecules other than the present anti-RGMa neutralizing antibody, such as nonpeptidic polymers, e.g. polyethylene glycol (PEG), radioactive substances, toxins, low molecular weight compounds, cytokines, growth factors *(e.g.* TGF-β, NGF, and neurotrophin), albumins, enzymes, and other antibodies.

The terms "F(ab')₂" and "Fab" mean antibody fragments produced by treating an immunoglobulin with pepsin or papain which are proteases, namely produced by digestion at the upstream or downstream side of the disulfide bond existing between two heavy chains in the hinge region. For example, when IgG is treated with papain, it is cleaved at the upstream side of the disulfide bond existing between the two heavy chains in the hinge region to produce two homologous antibody fragments each comprising a light chain comprising a VL (light chain variable region) and a CL (light chain constant region) and a heavy chain fragment comprising a VH (heavy chain variable region) and a CHγ1 (γ1 region in the heavy chain constant region), in which the light chain and the heavy chain fragment are linked to each other via a disulfide bond at the C-terminal domain. Each of the two homologous antibody fragments is referred to as Fab. Meanwhile, when IgG is treated with pepsin, it is cleaved at the downstream side of the disulfide bond existing between the two heavy chains in the hinge region to produce an antibody fragment that is slightly larger than the two Fab linked to each other at the hinge region. This antibody fragment is referred to as F(ab')₂.

### <Chimeric Antibody>

In a preferred mode of the anti-RGMa neutralizing antibody of the present invention, there is a chimeric antibody. Examples of the "chimeric antibody" include those in which the variable regions are derived from immunoglobulins from non-human animals (such as mouse, rat, hamster and chicken) and the constant regions are derived from human immunoglobulins. The chimeric antibody can be prepared, for example, by immunizing a mouse with the antigen, cleaving out a variable region that binds to the antigen from the gene encoding the mouse monoclonal antibody, and combining the variable region with a constant region of an antibody derived from human bone marrow. The constant region derived from a human immunoglobulin has a unique amino acid sequence depending on each isotype, such as IgG (IgG1, IgG2, IgG3, or IgG4), IgM, IgA (IgA1, or IgA2), IgD, or IgE. The constant region of the recombinant chimeric antibody according to the present invention may be a constant region of a human immunoglobulin belonging to any of the isotypes. The constant region is preferably a constant region of human IgG. The chimeric antibody gene thus prepared can be used to prepare an expression vector. A host cell is transformed with the expression vector to obtain a transformed cell that produces the chimeric antibody. Subsequently, the transformed cell is cultured to obtain the desired chimeric antibody from the culture supernatant.

### <Humanized Antibody>

In another preferred mode of the anti-RGMa neutralizing antibody of the present invention, there is a humanized antibody. The "humanized antibody" according to the present invention is an antibody obtained by grafting only the DNA sequence of the antigen binding sites (CDRs; complementarity determining regions) of an antibody derived from a nonhuman animal such as mouse to a human antibody gene (CDR grafting). The humanized antibody can be prepared according to the methods described in, for example, Japanese Translated PCT Patent Application Laid-open No. 1992-506458 and Japanese Patent No. 2912618. Specifically, the humanized antibody comprises CDRs partly or wholly derived from monoclonal antibodies from non-human mammals (such as mouse, rat, and hamster); framework regions of variable regions derived from human immunoglobulins; and constant regions derived from human immunoglobulins.

The humanized antibody according to the present invention can be produced, for example, as described below. Needless to say, however, the production method is not limited thereto.

For example, a recombinant humanized antibody derived from a mouse monoclonal antibody can be produced by genetic engineering with reference to Japanese Translated PCT Patent Application Laid-open No. 1992-506458 and Japanese Laid-open Patent Application (Kokai) No. 1987-296890. Specifically, DNA encoding the region of CDRs of a mouse heavy chain and DNA encoding the region of CDRs of a mouse light chain are isolated from hybridomas producing a mouse monoclonal antibody. Further, a human heavy chain gene encoding the whole region other than CDRs of the human heavy chain and a human light chain gene encoding the whole region other than CDRs of the human light chain are isolated from a human immunoglobulin gene.

The isolated DNA encoding the region of CDRs of the mouse heavy chain is grafted to the human heavy chain gene, and the product is introduced into an appropriate expression vector so that it is expressible. Similarly, the DNA encoding the region of CDRs of the mouse light chain is grafted to the human light chain gene, and the product is introduced into another appropriate expression vector so that it is expressible. Alternatively, the human heavy and light chain genes to which mouse CDRs are grafted may be introduced into the same expression vector so that they are expressible. A host cell is transformed with the expression vector thus prepared to obtain a transformed cell producing the humanized antibody. Subsequently, the transformed cell is cultured to obtain the desired humanized antibody from the culture supernatant.

### <Human Antibody>

In another preferred mode of the anti-RGMa neutralizing antibody of the present invention, there is a human antibody. The term "human antibody" refers to an antibody in which the entire region including heavy chain variable regions and heavy chain constant regions and light chain variable regions and light chain constant regions constituting the immunoglobulin are derived from genes encoding human immunoglobulins. Human antibodies can be prepared by introducing human antibody genes into mice. Human antibodies can be produced in the same manner as the above-described method for preparing polyclonal antibodies or monoclonal antibodies. Specifically, for example, the method comprises introducing at least human immunoglobulin genes into gene loci of a non-human mammal such as mouse to prepare a transgenic animal and immunizing the transgenic animal with an antigen.

For example, a transgenic mouse that produces a human antibody can be prepared according to the methods described, for example, in Nature Genetics, Vol. 7, p.13-21, 1994; Nature Genetics, Vol. 15, p.146-156, 1997; Japanese Translated PCT Patent Application Laid-open Nos. 1992-504365 and 1995-509137; WO 94/25585; Nature, Vol. 368, p.856-859, 1994; and Japanese Translated PCT Patent Application Laid-open No. 1994-500233. More specifically, for example, HuMab (registered trademark) Mouse (Medarex, Princeton, NJ), KMTM mouse (Kirin Pharma Company, Japan), and KM (FCyRIIb-KO) mouse may be used.

Specific examples of the anti-RGMa neutralizing antibody of the present invention include those having CDRs comprising specific amino acid sequences in the heavy chain variable region, and CDRs comprising specific amino acid sequences in the light chain variable region (preferably the anti-RGMa neutralizing antibodies (a) to (l) described above).

The amino acid sequence of the anti-RGMa neutralizing antibody (preferably the anti-RGMa neutralizing antibodies (a) to (l) described above) may have substitution, deletion, addition or insertion of one or several (1 to 20, 1 to 10, or 1 to 5, but preferably 1 or 2) amino acids as long as the antibody of the present invention maintains the characteristics of having an ability to bind to RGMa and inhibiting (neutralizing) the activity of RGMa. The substitution, deletion, or addition may be introduced in CDRs ,but it is preferably introduced in a region other than CDR. The amino acid substitution is preferably conservative substitution in order to maintain the characteristics of the present invention.

When the amino acid sequence of the anti-RGMa neutralizing antibody of the present invention (preferably the anti-RGMa neutralizing antibodies (a) to (l) described above) has a substitution, deletion, or the like therein, the amino acid sequence of the heavy chain variable region after the modification of the amino acid sequence has a % identity of 90% or more (more preferably 95%, 96%, 97%, 98%, or 99% or more) to the amino acid sequence before the modification, and the amino acid sequence of the light chain variable region after the modification of the amino acid sequence has a % identity of 90% or more (more preferably 95%, 96%, 97%, 98%, or 99% or more) to the amino acid sequence before the modification.

In the present invention the term "siRNA" refers to a short double strand RNA capable of inhibiting the expression of a target gene (a RGMa gene in the present invention). The nucleotide sequence and the length (base length) are not particularly limited as long as the function as an siRNA to inhibit the RGMa activity in the present invention is maintained. The base length is preferably less than about 30 bases, more preferably about 19 bases to 27 bases, and still more preferably about 21 bases to 25 bases.

In the present invention the term "shRNA" refers to a molecule with about 20 or more base pairs, which is a single strand RNA comprising a part having a palindromic nucleotide sequence to form a double-stranded structure in the molecule, and has a short hairpin structure with a 3'-terminal overhang. Such an shRNA can be introduced into a cell and then degraded into a length of about 20 bases (typically, for example, 21, 22, or 23 bases) in the cell to inhibit the expression of a target gene similarly to siRNA.

The siRNA and shRNA in the present invention may be in any form as long as they can inhibit the expression of the RGMa gene.

In the present invention, an siRNA or shRNA can be chemically synthesized by an artificial means. Antisense and sense RNAs can also be synthesized *in vitro* from DNA templates using, for example, a T7 RNA polymerase and a T7 promoter. The antisense oligonucleotide may be any nucleotide that is complementary to, or hybridizes to a consecutive nucleotide sequence having a length from 5 to 100 in the DNA sequence of a RGMa gene, and may be DNA or RNA. The antisense oligonucleotide may be modified insofar as its function is not impaired. The antisense oligonucleotide can be synthesized by a conventional method, and for example, it can be easily synthesized with a commercially available DNA synthesizer.

A preferred sequence can be selected by a common selection method, and the validation as the siRNA or shRNA according to the present invention can be made by evaluating inhibition of the expression of a functional RGMa.

### <Diabetic Dementia>.

Diabetic dementia in the present invention is dementia caused by glucose metabolism abnormality, and dementia associated with diabetes is a typical example. As for the pathogenic mechanism thereof, it is presumed that promotion of age-related change due to glucose toxicity, oxidative stress and/or advanced glycation end-product (AGE), etc., and further hyperinsulinemia, insulin resistance and insulin signal transduction failure are involved in addition to vascular factors such as cerebral infarction and atherosclerosis (Lancet Neurol, 5: 64-74, 2006). With respect to diabetic dementia, it is clinically indicated or suggested to have close relationship with glucose metabolism abnormality, while this dementia shows no, or only little pathological changes peculiar to Alzheimer's disease, or vascular lesions. Such diabetic dementia also includes dementia involving neurological impairment due to glucose metabolism disorders (including hyperglycemia, etc.). The diabetic dementia in the present invention also includes diabetic cognitive function disorders that are mild cognitive impairment before dementia.

### <Vascular Dementia>

Vascular dementia in the present invention means dementia that is caused by cerebral vascular disorders, and has a cause-and-effect relationship between cerebral vascular disorders and dementia. Causes of vascular dementia include disease types such as cerebral vascular insufficiency, hypoperfusion, and/or white matter lesion, in addition to cerebral infarction, cerebral hemorrhage, and/or subarachnoid hemorrhage. The vascular dementia in the present invention also includes vascular cognitive function disorders, that are mild cognitive impairment before dementia. The vascular dementia of the present invention can also be specified according to diagnostic criteria for vascular dementia (Non Patent Document 8).

The therapeutic objective (preferably a mammal, especially a human) in the present invention is a patient who has developed diabetic dementia or vascular dementia, and an agent for preventing or treating dementia selected from diabetic dementia and vascular dementia of the present invention can be administered to such a patient.

In this regard, a "treatment" includes any treatment of a disease in a therapeutic objective, preferably a mammal, and especially a human, and includes prevention of progression of a disease or symptom so as to extinguish, heal, alleviate, or mitigate such a disease and symptom.

The term "prevention" also includes prevention or inhibition of the onset of the above diseases in a therapeutic objective, preferably a mammal, and especially a human. Furthermore, "prevention" in the present invention includes "recurrence prevention" which prevents recurrence of the above-mentioned diseases in which remission and recurrence are repeated in a therapeutic objective, preferably a mammal, and especially a human.

### <Pharmaceutical composition>

The agent for preventing or treating dementia selected from diabetic dementia and vascular dementia in the present invention is usually administered systemically or locally in an oral or parenteral form.

The agent for preventing or treating dementia selected from diabetic dementia and vascular dementia in the present invention can be made into a formulation by blending a pharmaceutically acceptable carrier or additive as appropriate using a RGMa inhibiting substance as an active ingredient. The thus formulated pharmaceutical composition can be administered in an oral or parenteral form. Specifically, the agent can be formed into oral agents such as tablets, coated tablets, pills, powders, granules, capsules, solutions, suspensions, and emulsions; or parenteral agents such as injections, infusions, suppositories, ointments, and patches. The content ratio of the carrier or additive may be set as appropriate according to the ranges commonly used in the pharmaceutical field. There is no particular restriction on the carrier or additive to be blended, and examples thereof include water, physiological saline, other aqueous solvents, various carriers such as aqueous or oily bases; and various additives such as excipients, binders, pH adjusters, disintegrants, absorption promoters, lubricants, colorants, flavoring agents, and perfumes.

When the RGMa inhibiting substance is an anti-RGMa neutralizing antibody, a functionally modified antibody thereof, a conjugated antibody thereof, or an antigen-binding fragment thereof, the agent is preferably formulated into an injection or infusion with a pharmaceutically acceptable carrier, and administered via a parenteral route of administration, such as an intravenous, intramuscular, intradermal, intraperitoneal, subcutaneous, or local route.

The injection or infusion containing the anti-RGMa neutralizing antibody can be, for example, used as a solution, suspension, or emulsion. Examples of the solvent therefor include distilled water for injection, physiological saline, a glucose solution, and an isotonic solution (for example, solutions of sodium chloride, potassium chloride, glycerin, mannitol, sorbitol, boracic acid, borax, and propylene glycol).

The injections or infusions containing the anti-RGMa neutralizing antibody may further contain a stabilizer, a solubilizer, a suspending agent, an emulsifier, a soothing agent, a buffer agent, a preservative, an antiseptic, a pH adjuster, etc.

Examples of the stabilizer include albumin, globulin, gelatin, mannitol, glucose, dextran, ethylene glycol, propylene glycol, ascorbic acid, sodium bisulfite, sodium thiosulfate, sodium EDTA, sodium citrate, and dibutylhydroxytoluene.

Examples of the solubilizer include alcohols (such as ethanol), polyalcohols (such as propylene glycol and polyethylene glycol), and nonionic surfactants (such as polysorbate 80 (registered trademark), and HCO-50).

Examples of the suspending agent include glyceryl monostearate, aluminum monostearate, methylcellulose, carboxymethylcellulose, hydroxymethylcellulose, and sodium lauryl sulfate.

Examples of the emulsifier include gum arabic, sodium alginate, and tragacanth.

Examples of the soothing agent include benzylalcohol, chlorobutanol, and sorbitol.

Examples of the buffer agent include a phosphate buffer, acetate buffer, borate buffer, carbonate buffer, citrate buffer, and Tris buffer.

Examples of the preservative include methyl para-hydroxybenzoate, ethyl *para*-hydroxybenzoate, propyl *para*-hydroxybenzoate, butyl *para*-hydroxybenzoate, chlorobutanol, benzyl alcohol, benzalkonium chloride, sodium dehydroacetate, sodium edetate, boric acid, and borax.

Examples of the antiseptic include benzalkonium chloride, *para*hydroxybenzoic acid, and chlorobutanol.

Examples of the pH adjuster include hydrochloric acid, sodium hydroxide, phosphoric acid, and acetic acid.

When the RGMa inhibiting substance is a nucleic acid (such as siRNA, shRNA, and antisense oligonucleotide), it can be administered in the form of a nonviral or viral vector. When it is in the form of a nonviral vector, a method of introducing a nucleic acid molecule using a liposome (such as a liposome method, an HVJ-liposome method, a cationic liposome method, a lipofection method, or a lipofectamine method), a microinjection method, a method of transferring a nucleic acid molecule with a carrier (a metal particle) into a cell using a gene gun or the like, can be used. When siRNA or shRNA is administered to a living organism using a viral vector, a viral vector such as a recombinant adenovirus or retrovirus can be used. A DNA that expresses the siRNA or shRNA is introduced to a DNA virus or RNA virus such as detoxified retrovirus, adenovirus, adeno-associated virus, herpesvirus, vaccinia virus, poxvirus, poliovirus, sindbis virus, Sendai virus, or SV40. By infecting a cell or a tissue with the recombinant virus, the gene can be introduced into the cell or tissue.

The formulation thus obtained can be administered to, for example, a human or another mammal (such as a rat, mouse, rabbit, sheep, pig, cattle, cat, dog, or monkey) at an effective dose to prevent or treat dementia selected from diabetic dementia and vascular dementia. The dose is set as appropriate depending on the purpose, the severity of a disease, the age, weight, sex, and past history of a patient, the type of an active ingredient, or the like. For example, when the active ingredient is an anti-RGMa neutralizing antibody, the dose for an average human having a weight of about 65 kg to 70 kg is preferably about 0.02 mg to 4000 mg per day, more preferably about 0.1 mg to 200 mg per day. The total dose per day may be administered in a single dose or in divided doses.

### <Combination with Other Drug or Treatment>

In the present invention, an agent for preventing or treating dementia selected from diabetic dementia and vascular dementia may be administered in combination with an antidiabetic drug. A therapeutic agent for the antidiabetic drug to be used in combination includes, for example, an insulin resistance improving drug.

An agent for preventing or treating dementia selected from diabetic dementia and vascular dementia in the present invention may be administered in combination with an antithrombotic therapy or an antihypertensive agent. Examples of the antihypertensive agent to be used in combination include an angiotensin II receptor antagonist (such as candesartan), and an ACE inhibitor (such as perindopril).

The above other drug or treatment may be administered or performed before or after administration of an agent for preventing or treating dementia selected from diabetic dementia and vascular dementia according to the present invention, or may be administered or performed at the same time.

### EXAMPLES

The present invention will be described in more detail below with reference to Examples, which do not limit the scope of the present invention. As the anti-RGMa neutralizing antibody, an anti-RGMa neutralizing antibody comprising the amino acid sequences of (a) (SEQ ID NOS; 5 to 10) described herein was used in each example.

### [Example 1]

The therapeutic effect of an anti-RGMa neutralizing antibody on cognitive function impairment was examined using drug-induced type 1 diabetes model mice by a novel object recognition test.

### <Induction of Diabetes>

Female 7 to 8 week-old C57BL/6J mice were used in the experiment. To a diabetes-induced group, a 20 mg/mL streptozotocin (STZ: Sigma-Aldrich Co. LLC, St. Louis, MO, USA) was administered intraperitoneally at a single dose of 10 mL/kg, and to a non-diabetes-induced group, a vehicle was administered at a dose of 10 mL/kg. According to the method described in References 1 and 2, the blood glucose levels were measured 1 week after the diabetes induction. Individuals whose blood glucose level was less than 300 mg/dL were excluded.

### <Grouping, and Schedule of Antibody Administration >

Purchased mice were divided into two groups of "diabetes-anti-RGMa neutralizing antibody-administered group", and "diabetes-isotype control antibody (palivizumab) administered-group". Administration of the antibody to both the groups was started on day 3 after the diabetes induction. Each antibody was adjusted to a concentration of 6 mg/mL, and administered into the tail vein at a dose of 30 mg/kg once a week for total four times. Fourteen mice for the "diabetes-anti-RGMa neutralizing antibody-administered group", and fifteen mice for the "diabetes-isotype control antibody (palivizumab)-administered group" were used for the object recognition test. Ten mice for the "diabetes-anti-RGMa neutralizing antibody-administered group" and eight mice for the "diabetes-isotype control antibody (palivizumab)-administered group" respectively satisfying the criteria of the object recognition test were used for analysis. Meanwhile, seven non-diabetes mice (healthy mice) were used for calculating a DI value.

### <Novel Object Recognition Memory Test>

Acclimation (handling) was performed for 3 days, and on the next day each individual was put into an open field and allowed to go to free exploration for 15 minutes for acclimation to the field (acclimating trial 1). An acquisition trial was performed from the next day. Prior to this acquisition trial, each individual was allowed to go to free exploration in the field for 5 minutes (acclimating trial 2). After the acclimating trial 2, each individual was temporarily returned to a residence cage, and identically shaped objects (objects A) made from blocks were placed at two locations in the upper left and lower right corners on a diagonal of the field, and then each individual was returned to the field for free exploration (acquisition trial). After a 12 hour interval (decided by prior investigation of the conditions) following the completion of the acquisition trial, a test trial was executed. In executing the test trial, a bottle containing opaque beads (object B) was placed as a novel object in place of the object A having been placed in the lower right corner on the field diagonal. Meanwhile, the object A in the upper left corner on the field diagonal was placed in the same way as in the acquisition trial. In the test trial, each individual was returned to the field, where the objects A and B were placed, and allowed to go to free exploration for 10 minutes. Meanwhile, the object A and the object B were produced according to Reference 3. All the trials were recorded with a video camera, and the exploration time of each individual with respect to both objects in each trial was calculated after the behavioral test. In the exploratory behavior, odor-sniffing behaviors and object-touching behaviors to each object were included, but object-climbing behaviors were excluded. In the analysis, a Discrimination Index (DI) for the test trial, calculated with reference to Reference 4, was used. The DI is defined as [(exploration time to object B) - (exploration time to object A)] / [(exploration time to object B) + (exploration time to object A)]. An individual whose total exploration time to the objects A and B in the test trial was less than 30 seconds was not used in the analysis. The DI of the reference healthy mice was calculated by conducting this behavioral test with 16 week-old non-diabetic mice.

### <Results>

The Discrimination Index (DI) values obtained from the anti-RGMa neutralizing antibody-administered group and the isotype control antibody-administered group after the onset of pathological conditions of diabetes, as well as the DI value of healthy mice are shown in Fig. 1. In the "diabetes-isotype control antibody-administered group", significant decrease in the DI compared to the healthy mice was observed (p < 0.05, Tukey's multiple comparisons test). On the other hand, in the "diabetes-anti-RGMa neutralizing antibody-administered group", significant increase in the DI compared to the "diabetes-isotype control antibody-administered group" was observed (p < 0.05, Tukey's multiple comparisons test), while there was no difference compared to the healthy mice (p = 0.8640, Tukey's multiple comparisons test). From these results, it has been revealed that the object memory impaired by diabetes can be ameliorated by administration of an anti-RGMa neutralizing antibody.

From the above results, it has become clear that the RGMa inhibiting substance, especially the anti-RGMa neutralizing antibody exhibits therapeutic effect on diabetic dementia.

### [Example 2]

Impaired neurogenesis in the hippocampus has been suggested as a cause of hippocampal-dependent cognitive dysfunction in animal models of diabetes mellitus (Reference 6). The therapeutic effect of an anti-RGMa neutralizing antibody on the suppression of neurogenesis of the hippocampal dentate gyrus caused by diabetes was investigated using drug-induced type 1 diabetes model mice by an immunohistochemical staining method.

### <Induction of Diabetes>

Female 7 to 8 week-old C57BL/6J mice were used in the experiment. To a diabetes-induced group, a 20 mg/mL streptozocin (STZ: Sigma-Aldrich Co. LLC, St. Louis, MO, USA) was administered intraperitoneally at a single dose of 10 mL/kg, and to a non-diabetes-induced group, a vehicle was administered at a dose of 10 mL/kg. According to References 1 and 2, the blood glucose levels were measured 1 week after the diabetes induction. Individuals whose blood glucose level was less than 300 mg/dL were excluded.

### <Administration of Antibody>

Purchased mice were divided into four groups of a "non-diabetes-anti-RGMa neutralizing antibody-administered group", a "non-diabetes-isotype control antibody (palivizumab)-administered group", a "diabetes-anti-RGMa neutralizing antibody administered-group", and a "diabetes-isotype control antibody (palivizumab)-administered group". To the diabetes groups, a STZ solution was administered, and to the non-diabetes-induced groups, a vehicle was administered, and from three days later, administration of the anti-RGMa neutralizing antibody, or isotype control antibody was initiated. Each antibody was adjusted to a concentration of 6 mg/mL and then administered into the tail vein once a week each at a dose of 30 mg/kg for a total of six times.

### <Tissue Sampling and Immunohistochemical Staining>

A sample was taken from the mouse brain 6 weeks after diabetes induction. Under adequate anesthesia, perfusion fixation was conducted with 4% paraformaldehyde (PFA), then the brain was taken out and fixed in 4% PFA as post fixation. The tissues were then transferred to a 30% sucrose solution with PBS as the solvent, and left standing at 4°C for 2 days to 3 days, and then embedded in an OCT compound (Sakura Finetek USA Inc., Torrance, CA, USA). The brain was then sliced to a section with a thickness of 30 µm using a cryostat, and the prepared section was bonded to a MAS coated slide glass and then subjected to immunostaining. Blocking was performed with a PBS containing 3% Normal Donkey Serum (NDS), and 0.3% Triton X-100 (blocking solution) at room temperature for 1 hour, followed by washing with PBS. The sample was then made to react at 4°C overnight using an anti-mouse doublecortin antibody (1:100; Abcam, Cambridge, UK, diluted with a blocking solution) as a primary antibody. After washing with PBST, the reaction was further conducted using an Alexa Fluor 568 donkey antirabbit IgG (H+L) antibody (1:500; Invitrogen, Waltham, MA, USA, diluted with a blocking solution) as a secondary antibody at room temperature for 1 hour. Then, the sample was washed with PBST, then subjected to nuclear staining using DAPI (1 µg/mL), and encapsulated. The entire hippocampal dentate gyrus was imaged using a confocal laser scanning microscope FV3000 (Olympus, Tokyo, Japan), and the number of cell bodies of doublecortin-positive neural progenitor cells was counted. The measurement of the hippocampal dentate gyrus was conducted for 6 sections per each individual. The number of doublecortin positive cells was normalized based on the area of the hippocampal dentate gyrus measured using ImageJ software.

### <Results>

The numbers of doublecortin positive cells normalized based on the area of the hippocampal dentate gyrus are shown in Fig. 2. There was no change in the number of doublecortin positive cells among the non-diabetes-induced groups irrespective of the type of antibody administered (p > 0.9999, Tukey's multiple comparisons test). Meanwhile, the number of doublecortin positive cells in the "diabetes-isotype control antibody-administered group" was significantly reduced compared to the "non-diabetes-anti-RGMa neutralizing antibody-administered group" or "non-diabetes-isotype control antibody-administered group" (vs. "non-diabetes-anti-RGMa neutralizing antibody-administered group", p < 0.001, Tukey's multiple comparisons test; and vs. "non-diabetes-isotype control antibody-administered group"; p < 0.001, Tukey's multiple comparisons test). On the other hand, the number of doublecortin positive cells was significantly improved in the "diabetes-anti-RGMa neutralizing antibody-administered group" compared to the "diabetes-isotype control antibody-administered group" (p < 0.05, Tukey's multiple comparisons test). The "diabetes-anti-RGMa neutralizing antibody-administered group" did not show a significant difference in the number of doublecortin positive cells compared to the "non-diabetes-isotype control antibody-administered group" and the "non-diabetes-anti-RGMa neutralizing antibody-administered group" (vs. "non-diabetes-isotype control antibody-administered group", p = 0.1071, Tukey's multiple comparisons test; and vs. "non-diabetes-anti-RGMa neutralizing antibody-administered group"; p = 0.1130, Tukey's multiple comparisons test). From these results, it has been revealed that decrease in neurogenesis by doublecortin-positive neural progenitor cells in the hippocampal dentate gyrus caused by diabetes can be improved by administration of an anti-RGMa neutralizing antibody.

From these results, it has become clear that a RGMa inhibiting substance, especially anti-RGMa neutralizing antibody, exhibits therapeutic effects on impaired neurogenesis in the hippocampus as one of the causes of diabetic dementia.

### [Example 3] Effect of Therapeutic Intervention with Anti-RGMa Neutralizing Antibody on Novel Object Recognition Memory

The therapeutic effect of an anti-RGMa neutralizing antibody on impairment of cognitive function in chronic cerebral hypoperfusion model mice induced by placement of micro-coils in the bilateral internal carotid arteries (bilateral common carotid artery stenosis model: BCAS model) was studied by means of a novel object recognition test.

### <Induction of chronic cerebral hypoperfusion model>

Male C57BL/6J mice aged 9 week-old to 10 week-old were used for the experiment. Under isoflurane inhalation anesthesia (4% induction, 1.5% maintenance), each mouse was immobilized in the prone position and the cerebral blood flow was measured by laser speckle flowmetry. During the measurement, the rectal temperature was controlled in a range of 35.0°C to 36.5°C using a heat pad. Thereafter the mouse was immobilized in the supine position, and the neck was incised, the carotid sheath was opened, and micro-coils were placed in the bilateral internal carotid arteries (Reference 5). The cerebral blood flow was measured 1 day after the BCAS induction, and individuals in which the cerebral blood flow has not decreased to 90% or less of the preoperative level were excluded. OZ-3 (Omegawave, Tokyo, Japan) was used for measuring the cerebral blood flow.

<Novel Object Recognition Memory Test> Each individual was put into an open field and allowed to go to free exploration for 15 min for acclimation to the field (acclimating trial 1). The next day, each individual was allowed to go to free exploration in the field for 5 min (acclimating trial 2) before an acquisition trial. After executing the acclimating trial 2, each individual was temporarily returned to a residence cage, and identically shaped objects (objects A) made from blocks were fixed using a double-faced tape at two locations in the upper left and lower right corners on a diagonal of the field off the wall by 10 cm, and then each individual was returned to the field for free exploration (acquisition trial). After a 12-hour intermission following the completion of the acquisition trial, a test trial was executed. In executing the test trial, a bottle containing opaque beads (object B) was fixed using a double-faced tape at a position off the wall by 10 cm as a novel object in place of the object A having been placed in the lower right corner on the field diagonal. Meanwhile, the object A in the upper left corner on the field diagonal was placed in the same way as in the acquisition trial. After the elapse of the intermission, each individual was returned to the field, where the objects A and B were placed, and allowed to go to free exploration for 10 min (test trial). Meanwhile, the object A and the object B used in each trial were produced according to prior research (Reference 3). During the interval, each individual was allowed to free access to both water and food in the residence cage. All the trials were recorded with a video camera, and the exploration time of each individual with respect to the objects in each trial was calculated after the behavioral test. In the exploratory behavior, odor-sniffing behaviors and object-touching behaviors to each object were included, but object-climbing behaviors were excluded. In the analysis, a Discrimination Index (DI) for the test trial was used. The DI is defined as [(exploration time to object B) - (exploration time to object A)] / [(exploration time to object B) + (exploration time to object A)]. An Individual whose total exploration time to the objects A and B in the test trial was less than 30 sec was not used in the analysis.

### <Antibody administration>

Purchased mice were divided into three groups of BCAS-anti-RGMa neutralizing antibody-administered group, BCAS-isotype control antibody (Palivizumab)-administered group, and sham-isotype control antibody (Palivizumab)-administered group. For all the three groups, administration of an antibody was started 3 days after the BCAS operation or Sham operation. Each antibody was adjusted to a concentration of 2 mg/mL, and administered intraperitoneally at a weight-dependent dose of 10 mg/kg twice weekly in total 7 doses. Number of animals in each group is as follows:
BCAS-anti-RGMa neutralizing antibody-administered group: 13
BCAS-isotype control antibody (Palivizumab)-administered group: 9
Sham-isotype control antibody (Palivizumab)-administered group: 9

### <Results>

There was a significant decrease in DI in the BCAS-isotype control antibody-administered group compared to Sham-isotype control antibody (Palivizumab)-administered group (p<0.001, Tukey's multiple comparison test). On the other hand, in the BCAS-anti-RGMa neutralizing antibody-administered group, DI was significantly increased compared to the BCAS-isotype control antibody-administered (p<0.001, Tukey's multiple comparison test). This indicates that the therapeutic intervention by administration of the anti-RGMa neutralizing antibody significantly ameliorates object recognition memory impairment caused by chronic ischemia (Fig. 3).

From these results, it has become clear that a RGMa inhibiting substance, especially anti-RGMa neutralizing antibody, exhibits therapeutic effects on vascular dementia.

### [References]

1. Deeds MC, Anderson JM, Armstrong AS, et al. Single dose streptozotocininduced diabetes: Considerations for study design in islet transplantation models, Lab Anim., 2011; 45(3): 131-140. doi:10.1258/la.2010.010090
2. O'brien PD, Sakowski SA, Feldman EL. Mouse models of diabetic neuropathy. ILAR J. 2014; 54(3): 259-272. doi:10.1093/ilar/ilt052
3. Leger M, Quiedeville A, Bouet V, et al. Object recognition test in mice. Nat Protoc. 2013; 8(12): 2531-2537. doi:10.1038/nprot.2013.155
4. Grinan-Ferre C, Puigoriol-Illamola D, Palomera-avalos V, et al. Environmental enrichment modified epigenetic mechanisms in SAMP8 mouse hippocampus by reducing oxidative stress and inflammaging and achieving neuroprotection. Front Aging Neurosci. 2016; 8(OCT). doi:10.3389/fnagi.2016.00241
5. Hattori Y et al, Gradual Carotid Artery Stenosis in Mice Closely Replicates Hypoperfusive Vascular Dementia in Humans. Journal of the American Heart Association 2016 2 Feb.22;5(2): e002757.DOI: 10.1161/JAHA.115.002757
6. Stranahan, A., Arumugam, T., Cutler, R. et al. Diabetes impairs hippocampal function through glucocorticoid-mediated effects on new and mature neurons. Nat Neurosci 11, 309-317 (2008).

### <Description of Sequence Listing>

SEQ ID NO: 1: Amino acid sequence of human RGMa precursor protein
SEQ ID NO: 2: Amino acid sequence of mouse RGMa precursor protein
SEQ ID NO: 3: Amino acid sequence of rat RGMa precursor protein
SEQ ID NO: 4: DNA sequence of human RGMa gene
SEQ ID NO: 5: Amino acid sequence of LCDR1 of anti-RGMa neutralizing antibody r116A3
SEQ ID NO: 6: Amino acid sequence of LCDR2 of anti-RGMa neutralizing antibody r116A3
SEQ ID NO: 7: Amino acid sequence of LCDR3 of anti-RGMa neutralizing antibody r116A3
SEQ ID NO: 8: Amino acid sequence of HCDR1 of anti-RGMa neutralizing antibody r116A3
SEQ ID NO: 9: Amino acid sequence of HCDR2 of anti-RGMa neutralizing antibody r116A3
SEQ ID NO: 10: Amino acid sequence of HCDR3 of anti-RGMa neutralizing antibody r116A3
SEQ ID NO: 11: Amino acid sequence of LCDR1 of anti-RGMa neutralizing antibody r70E
SEQ ID NO: 12: Amino acid sequence of LCDR2 of anti-RGMa neutralizing antibody r70E
SEQ ID NO: 13: Amino acid sequence of LCDR3 of anti-RGMa neutralizing antibody r70E
SEQ ID NO: 14: Amino acid sequence of HCDR1 of anti-RGMa neutralizing antibody r70E
SEQ ID NO: 15: Amino acid sequence of HCDR2 of anti-RGMa neutralizing antibody r70E
SEQ ID NO: 16: Amino acid sequence of human RGMa epitope
SEQ ID NO: 17: Amino acid sequence of LCDR1 of anti-RGMa neutralizing antibody 5F9
SEQ ID NO: 18: Amino acid sequence of LCDR2 of anti-RGMa neutralizing antibody 5F9
SEQ ID NO: 19: Amino acid sequence of LCDR3 of anti-RGMa neutralizing antibody 5F9
SEQ ID NO: 20: Amino acid sequence of HCDR1 of anti-RGMa neutralizing antibody 5F9
SEQ ID NO: 21: Amino acid sequence of HCDR2 of anti-RGMa neutralizing antibody 5F9
SEQ ID NO: 22: Amino acid sequence of HCDR3 of anti-RGMa neutralizing antibody 5F9
SEQ ID NO: 23: Amino acid sequence of LCDR1 of anti-RGMa neutralizing antibody 8D1
SEQ ID NO: 24: Amino acid sequence of LCDR2 of anti-RGMa neutralizing antibody 8D1
SEQ ID NO: 25: Amino acid sequence of LCDR3 of anti-RGMa neutralizing antibody 8D1
SEQ ID NO: 26: Amino acid sequence of HCDR1 of anti-RGMa neutralizing antibody 8D1
SEQ ID NO: 27: Amino acid sequence of HCDR2 of anti-RGMa neutralizing antibody 8D1
SEQ ID NO: 28: Amino acid sequence of HCDR3 of anti-RGMa neutralizing antibody 8D1
SEQ ID NO: 29: Amino acid sequence of LCDR1 of anti-RGMa neutralizing antibody AE12-1
SEQ ID NO: 30: Amino acid sequence of LCDR2 of anti-RGMa neutralizing antibody AE12-1
SEQ ID NO: 31: Amino acid sequence of LCDR3 of anti-RGMa neutralizing antibody AE12-1
SEQ ID NO: 32: Amino acid sequence of HCDR1 of anti-RGMa neutralizing antibody AE12-1
SEQ ID NO: 33: Amino acid sequence of HCDR2 of anti-RGMa neutralizing antibody AE12-1
SEQ ID NO: 34: Amino acid sequence of HCDR3 of anti-RGMa neutralizing antibody AE12-1
SEQ ID NO: 35: Amino acid sequence of LCDR3 of anti-RGMa neutralizing antibody AE12-1Y
SEQ ID NO: 36: Amino acid sequence of human RGMa epitope
SEQ ID NO: 37: Amino acid sequence of human RGMa epitope
SEQ ID NO: 38: Amino acid sequence of human RGMa epitope
SEQ ID NO: 39: Amino acid sequence of human RGMa epitope
SEQ ID NO: 40: Amino acid sequence of LCDR3 of anti-RGMa neutralizing antibody AE12-1F
SEQ ID NO: 41: Amino acid sequence of LCDR3 of anti-RGMa neutralizing antibody AE12-1H
SEQ ID NO: 42: Amino acid sequence of LCDR3 of anti-RGMa neutralizing antibody AE12-1L
SEQ ID NO: 43: Amino acid sequence of LCDR3 of anti-RGMa neutralizing antibody AE12-1V
SEQ ID NO: 44: Amino acid sequence of LCDR3 of anti-RGMa neutralizing antibody AE12-1I
SEQ ID NO: 45: Amino acid sequence of LCDR3 of anti-RGMa neutralizing antibody AE12-1K

### INDUSTRIAL APPLICABILITY

Since the RGMa inhibiting substance is useful for preventing or treating diabetic dementia or vascular dementia, the present invention has high utility in the pharmaceutical industry.

## Claims

1. An agent for preventing or treating dementia selected from diabetic dementia and vascular dementia comprising a RGMa inhibiting substance.

2. An agent for preventing or treating diabetic dementia comprising a RGMa inhibiting substance.

3. An agent for preventing or treating vascular dementia comprising a RGMa inhibiting substance.

4. The preventive or therapeutic agent according to any one of claims 1 to 3, wherein the RGMa inhibiting substance is an anti-RGMa neutralizing antibody.

5. The preventive or therapeutic agent according to claim 4, wherein the anti-RGMa neutralizing antibody is a humanized antibody.

6. The preventive or therapeutic agent according to claim 4 or 5, wherein the anti-RGMa neutralizing antibody is an antibody recognizing an amino acid sequence selected from SEQ ID NOS: 16, 36, 37, 38 and 39.

7. The preventive or therapeutic agent according to any one of claims 4 to 6, wherein the anti-RGMa neutralizing antibody is an antibody selected from the following (a) to (l):
(a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6, and an LCDR3 comprising the amino acid sequence represented by SEQ ID NO: 7, and a heavy chain variable region comprising an HCDR1 comprising the amino acid sequence represented by SEQ ID NO: 8, an HCDR2 comprising the amino acid sequence represented by SEQ ID NO: 9, and an HCDR3 comprising the amino acid sequence represented by SEQ ID NO: 10;
(b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12, and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15, and an HCDR3 comprising an amino acid sequence comprising SFG;
(c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18, and an LCDR3 comprising the amino acid sequence represented by SEQ ID NO: 19, and a heavy chain variable region comprising an HCDR1 comprising the amino acid sequence represented by SEQ ID NO: 20, an HCDR2 comprising the amino acid sequence represented by SEQ ID NO: 21, and an HCDR3 comprising the amino acid sequence represented by SEQ ID NO: 22;
(d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24, and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ ID NO: 27, and an HCDR3 comprising the amino acid sequence represented by SEQ ID NO: 28;
(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 42, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 43, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34; and
(l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34.

8. A method of preventing or treating dementia selected from diabetic dementia and vascular dementia, which comprises administration of an effective dose of a RGMa inhibiting substance to a mammal in need of treatment.

9. The preventive or therapeutic method according to claim 8, wherein the RGMa inhibiting substance is an anti-RGMa neutralizing antibody.

10. Use of a RGMa inhibiting substance in manufacture of an agent for preventing or treating dementia selected from diabetic dementia and vascular dementia.

11. The use according to claim 10, wherein the RGMa inhibiting substance is an anti-RGMa neutralizing antibody.
